# EUROPEAN PATENT APPLICATION

(11) **EP 2 113 285 A1**
(43) Date of publication of application: **04.11.2009**
(21) Application number: 08155360.4
(22) Date of filing: 29.04.2008
(51) Int. Cl.: A61P 25/28

(54) **Role of (de)acetylation in counteracting protein aggregation**

(71) Applicant: Rijksuniversiteit Groningen, 9712 CP Groningen (NL)
(72) Inventor: The designation of the inventor has not yet been filed
(74) Representative: Hatzmann, Martin

(57) **Abstract**

The invention provides a method for influencing an activity of a heat shock protein which is a member of the Hsp40/DnaJ family, the method comprising acetylating or deacetylating said heat shock protein.

## Description

The invention relates to the fields of biology and medicine.

Cellular protein aggregation is a process that acts in competition with normal protein folding processes. Proteins that are not correctly folded often aggregate and the presence of insoluble intracellular complexes result in a wide range of diseases, comprising, amongst other things, Alzheimer's disease, transmissible spongiform encephalopathies, Parkinson's disease, type 2 diabetes, transthyretin-mediated amyloid diseases such as familial amyloid cardiomyopathy and familial amyloidotic polyneuropathy, and amyotrophic lateral sclerosis.

A number of human genetic diseases are associated with an expansion of short tandem repeats in coding or non-coding gene regions. Examples comprise spinocerebellar ataxias type 10, which is associated with an expansion of a pentanucleotide repeat (ATTCT) in intron 9 of the SCA10 gene sequence to as many as 4500 copies; myotonic dystrophy type 1, an autosomal dominant multisystemic disorder characterized by the amplification of an unstable (CTG)n repeat in the 3'-untranslated region of a protein kinase gene; myotonic dystrophy type 2, characterized by expansion of a CCTG repeat in intron 1 of a zinc finger protein gene, and a group of neurodegenerative disorders or polyglutamine-mediated diseases, which are characterized by an expansion of a polyglutamine-encoding CAG repeat in a diversity of genes that are otherwise unrelated.

Protein misfolding and aggregation are also associated with cytotoxicity in polyglutamine diseases. Polyglutamine-mediated neurodegenerative disorders comprise to date a total of nine established neurodegenerative disorders, which are characterized by the genetic expansion of polyglutamine repeats in specified proteins. Clinical features and patterns of neuronal degeneration differ among the diseases but important pathogenic characteristics are common: all are progressive diseases characterized by neuronal dysfunction and neuronal loss progress over 10-30 years after onset, and are ultimately fatal. The underlying mechanism of polyglutamine-mediated neurotoxicity has not been fully elucidated. Expanded polyglutamine repeats are thought to result in conformational changes in the proteins that lead to misfolding, aggregation, inclusion body formation, and eventual neuronal cell death.

Polyglutamine-mediated neurodegenerative disorders comprise X-linked spinal and bulbar muscular atrophy (Kennedy disease), an X-linked recessive disorder caused by enlargement of a poly(Q) stretch in the androgen receptor protein; Huntington's disease, an autosomal dominant disorder caused by enlargement of a poly(Q) stretch in the huntingtin protein; dentatorubral-pallidoluysian atrophy (Haw River syndrome), an autosomal dominant disorder caused by enlargement of a poly(Q) stretch in the atrophin-1 protein; spinocerebellar ataxia type 1; an autosomal dominant disorder caused by enlargement of a poly(Q) stretch in the ataxin-1 protein; spinocerebellar ataxia type 2, an autosomal dominant disorder caused by enlargement of a poly(Q) stretch in the ataxin-2 protein; spinocerebellar ataxia type 3 (Machado-Joseph disease), an autosomal dominant disorder caused by enlargement of a poly(Q) stretch in the ataxin-3 protein; spinocerebellar ataxia type 6; an autosomal dominant disorder caused by enlargement of a poly(Q) stretch in a voltage-dependent calcium channel subunit; spinocerebellar ataxia type 7, an autosomal dominant disorder caused by enlargement of a poly(Q) stretch in the ataxin-7 protein; and spinocerebellar ataxia type 17, an autosomal dominant disorder caused by enlargement of a poly(Q) stretch in a TATA binding protein.

The expansion of a polyglutamine-encoding CAG repeat results in the extension of a stretch of glutamines in the encoded proteins from between about 4-40 residues to between about 20-100 residues, whereby a pathological threshold depends on the neurodegenerative disorder. The age of onset of clinical manifestations is inversely correlated to the length of the polyglutamine expansion. Proteins with an enlarged stretch of polyglutamines tend to aggregate and neuronal intranuclear inclusions comprising such aggregates are found in distinct neuronal populations in diseased individuals, resulting in dysfunctionality and degeneration of the affected neurons. The identity of the affected neuronal populations is depending on the disorder. For example, in X-linked spinal and bulbar muscular atrophy lower motor neurons are primarily affected, resulting in progressive bulbar and proximal limb muscle weakness and atrophy (Wood et al. (2003) Neuropathology and Applied Neurobiology 29: 529-545).

Polyglutamine diseases are diseases of misfolding, in which the disease-related proteins are prone to aggregation. Insoluble intracellular protein aggregates are hallmarks of these disorders. This suggests that chaperones, the Ubiquitin Proteasome System (UPS) and other protein degradation systems could play a significant role in protection against the disease progression. Indeed, genes involved in RNA metabolism, protein synthesis, protein folding (such as chaperones), protein trafficking, regulators of the oxidative stress and components of the proteasome have been identified in screenings for modifiers of polyglutamine aggregation in C. elegans (Nollen et al., 2004) or neurodegeneration in Drosophila (Fernandez-Funez et al., 2000; Kazemi-Esfarjani and Benzer, 2000).

Molecular chaperones are a group of structurally diverse, evolutionary highly conserved proteins that interact with the non-native conformation of other proteins and mediate their folding or assembly, but are not components of the final functional structures (Frydman, 2001; Hartl and Hayer-Hartl, 2002). Chaperones are ubiquitously expressed and are found in all cellular compartments of the eukaryotic cell, which reflects their essential function under normal growth conditions. Despite their similar role in facilitating folding and assembly of proteins, some of their specific functions differ, and in many cases they act in tandem with eachother (Hartl and Hayer-Hartl, 2002). A large number of studies have been performed in order to determine the role of molecular chaperones in the pathogenesis of expanded polyglutamine proteins. Indeed, there are several reports that members of the Heat Shock Protein 70 (Hsp70) family and/or its cofactors can modulate polyglutamine aggregation and pathogenesis. Whereas in most cell models the Hsp70 machine reduces the extent of polyglutamine aggregation, it is still unclear what the crucial factors are.

Human molecular chaperones comprise Hsp 110, Hsp 70, and Hsp 40 heat shock protein family members, whereby each family is characterized by the presence of specific protein domains. The Hsp 110 family comprises 3 members, the Hsp 70 family comprises 11 members, whilst the Hsp 40 family comprises more than 40 members (see Table 1). Many studies have been performed in order to determine the role of molecular chaperones in protein aggregation events. However, which heat shock family members are effective in counteracting protein aggregation clearly differs between models. Furthermore, there are large differences in the efficacy of different kinds of molecular chaperones of counteracting protein aggregation and *in vitro* results are often not reproducible *in vivo*. For example, while components of the Hsp70 machine can reduce aggregation and pathogenesis in polyglutamine disease, the magnitude of effects, especially for Huntington's disease has been moderate. Also, no clear insight has been obtained on how the Hsp70 handles the polyglutamine-containing proteins. Furthermore, there are several indications that polyglutamine containing proteins are very poor substrates for the proteasome (Venkatraman et al., 2004; Holmberg et al., 2004).

Recently, we have disclosed that DnaJB8, a member of the Hsp40 family, is a particularly effective chaperone capable of counteracting protein aggregation and toxicity mediated by protein aggregation (International patent application PCT/NL2008/050207). Furthermore, the chaperone DnaJB6 is also effectively capable of counteracting protein aggregation and toxicity mediated by protein aggregation. An advantage of DnaJB6 is that it is ubiquitously present in mammals. In view of the activities of DnaJB8 and DnaJB6, a substance that is capable of enhancing the amount and/or activity of DnaJB8 and/or DnaJB6, or a functional part, derivative and/or analogue thereof, is particularly suitable for counteracting protein aggregation. It is an object of the present invention to provide such substances. It is a further object to provide alternative means and methods for counteracting diseases associated with protein aggregation, and to identify substances capable of counteracting such diseases. Moreover, it is a further object to provide alternative means and methods for improving the activity of other heat shock proteins.

The present invention provides the insight that the activity of heat shock proteins which are members of the Hsp40/DnaJ family is influenced by acetylation and/or deacetylation. Acetylation and deacetylation are known to play a role in other biological processes. For instance, histone acetyltransferases (HATs) and histone deacetylases (HDACs) are enzymes that catalyze the acetylation and deacetylation, respectively, of proteins at lysine residues. Although originally discovered as histone modification it is nowadays known that many proteins can be post-translationally modified by (de)acetylation which may drastically affect protein stability and function, including that of Hsp90 (Purva Bali, Michael Pranpat, James Bradner, Maria Balasis, Warren Fiskus, Fei Guo, Kathy Rocha, Sandhya Kumaraswamy, Sandhya Boyapalle, Peter Atadja, Edward Seto, and Kapil Bhalla, Inhibition of Histone Deacetylase 6 Acetylates and Disrupts the Chaperone Function of Heat Shock Protein 90, J. Biol. Chem., 280 (2005) 26729-26734). Until the present invention it was not known that the activity of heat shock proteins which are members of the Hsp40/DnaJ family is also influenced by acetylation and/or deacetylation. Interestingly, acetyltransferase activity is reduced in cell culture models of Huntington's disease (Nucifora FC Jr, Sasaki M, Peters MF, Huang H, Cooper JK, Yamada M, Takahashi H, Tsuji S, Troncoso J, Dawson VL, Dawson TM, Ross CA (2001) Interference by huntingtin and atrophin-1 with CBP1-mediated transcription leading to cellular toxicity. Science 291:2423-2428) and HDAC inhibition or downregulation of individual *C. elegans* HDACs enhanced mutant huntingtin toxicity, with the exception of knockdown of *C. elegans* HDAC-3 that suppressed toxicity (E. A. Bates, M. Victor, A.K. Jones, Y. Shi, and A. C. Hart, Differential Contributions of Caenorhabditis elegans Histone Deacetylases to Huntingtin Polyglutamine Toxicity. The Journal of Neuroscience, 26, 2006, 2830-2838). Moreover, it is already known for some years that the NAD-dependent deacetylase Sir-2, that is activated after calory restriction, not only plays a crucial role life span but also affects stress resistance towards heat shock and neurodegeneration evoked by protein folding diseases (see for review: L. Guarente and F. Picard, Calorie Restrietion- the SIR2 Connection, Cell 120 (2005) 473-482). More specifically, activation of Sir-2 rescued early neuronal dysfunction phenotypes induced by mutant polyglutamines in transgenic *Caenorhabditis elegans* (Parker JA, Arango M, Abderrahmane S, Lambert E, Tourette C, Catoire H, Néri C. Resveratrol rescues mutant polyglutamine cytotoxicity in nematode and mammalian neurons, Nat Genet. 37 (2005) 349-350). Since Sir-2 has many different substrates (Guarente and F. Picard, Calorie Restriction- the SIR2 Connection, Cell 120 (2005) 473-482) it was until the present invention unknown by what mechanism it may affect these various endpoints.

Hsp 40/DnaJ family members are homologous to the *Escherichia coli* DnaJ protein and contain a characteristic J-domain that mediates interaction with Hsp 70 and regulate ATPase activity by Hsp 70. Members of the Hsp40/DnaJ family are capable of counteracting protein aggregation. As described in PCT/NL2008/050207, two Hsp40/DnaJ members which are particularly well capable of counteracting protein aggregation are DnaJB6 and DnaJB8.
As used herein, heat shock proteins which are members of the Hsp40/DnaJ family are also called "Hsp40/DnaJ heat shock proteins" or "Hsp40/DnaJ proteins".

Now that the present invention provides the insight that the activity of Hsp40/DnaJ heat shock proteins is influenced by acetylation and/or deacetylation, it has become possible to influence their activity. One embodiment therefore provides a method for influencing an activity of a heat shock protein which is a member of the Hsp40/DnaJ family, the method comprising acetylating or deacetylating said heat shock protein. This is preferably done with an acetylase or deacetylase. One embodiment therefore provides a method for influencing an activity of a heat shock protein which is a member of the Hsp40/DnaJ family, the method comprising contacting said heat shock protein with an acetylase or deacetylase.
As used herein, an acetylase comprises a compound which is capable of acetylating a substrate. A deacetylase comprises a compound capable of deacetylating a substrate. Said compound preferably comprises a protein or a (poly)peptide.
Of course, it is also possible to use a nucleic acid molecule encoding an acetylase and/or a deacetylase. After administration of such nucleic acid to a cell, the cell's machinery will translate the encoded acetylase and/or deacetylase. After said acetylase and/or deacetylase has been produced, it will influence the activity of a Hsp40/DnaJ heat shock protein.
In yet another embodiment, a compound capable of enhancing the amount and/or activity of an acetylase and/or a deacetylase in a cell is used. This way, an activity of a Hsp40/DnaJ heat shock protein is indirectly influenced. Administration of said compound results in an increased amount and/or activity of an acetylase and/or deacetylase. As a result, the (overall) effect of said acetylase and/or deacetylase upon a Hsp40/DnaJ heat shock protein is enhanced. Said compound capable of enhancing the amount and/or activity of an acetylase and/or deacetylase for instance comprises a compound capable of enhancing expression of said acetylase or deacetylase. Alternatively, or additionally, said compound comprises an activator of at least one acetylase or deacetylase.

Now that the effect of acetylases and deacetylases on the activity of Hsp40/DnaJ heat shock proteins is known, such acetylase and/or deacetylase is in a preferred embodiment used as a medicament or prophylactic agent for influencing an activity of a Hsp40/DnaJ heat shock protein. Alternatively, as explained above, a nucleic acid molecule encoding an acetylase and/or a deacetylase, or a compound capable of enhancing the amount and/or activity of an acetylase and/or a deacetylase, is used. According to the present invention, a deacetylase is preferably used for enhancing the anti-protein aggregation activity of a Hsp40/DnaJ heat shock protein. Hence, a deacetylase, a nucleic acid molecule encoding a deacetylase and/or a compound capable of enhancing the amount and/or activity of a deacetylase is particularly suitable for use as a medicament or prophylactic agent. One embodiment therefore provides a deacetylase or a nucleic acid molecule encoding a deacetylase or a compound capable of enhancing the amount and/or activity of a deacetylase in a cell, for use as a medicament or prophylactic agent for influencing an activity of a heat shock protein which is a member of the Hsp40/DnaJ family. Of course, the use of these compounds for the preparation of a medicament is also herewith provided. One embodiment thus provides a use of a deacetylase or a nucleic acid molecule encoding a deacetylase or a compound capable of enhancing the amount and/or activity of a deacetylase in a cell, for the preparation of a medicament or prophylactic agent for influencing an activity of a heat shock protein which is a member of the Hsp40/DnaJ family. Preferably, said activity of said heat shock protein is enhanced. Said heat shock protein is preferably capable of at least in part counteracting and/or preventing a disorder associated with protein aggregation, so that said disorder is particularly well counteracted and/or prevented by increasing the activity of said heat shock protein.
A disorder associated with protein aggregation is defined as a disorder that is characterized by an accumulation of aggregated protein, such as for instance Alzheimer's disease and transmissible spongiform encephalopathies, Parkinson's disease, type 2 diabetes, transthyretin-mediated amyloid diseases, amyotrophic lateral sclerosis (Lou Gehrig's Disease), and diseases characterized by expansions of a small nucleotide repeat, including but not limited to Friedreich's ataxia, Myotonic dystrophy types 1 and 2, and polyglutamine-mediated disorders.

In a preferred embodiment, a deacetylase, nucleic acid molecule, compound or use according to the invention is provided wherein said disorder is associated with polyglutamine-mediated protein aggregation. Polyglutamine-mediated neurodegenerative disorders are described hereinbefore.
In a particularly preferred embodiment, the invention provides a deacetylase, nucleic acid molecule, compound or use according to the invention wherein said disorder is selected from Huntington's disease, dentatorubral-pallidoluysian atrophy, X-linked spinal and bulbar muscular atrophy, and spinocerebellar ataxias (SCA).

DnaJB6 and DnaJB8 are members of the Hsp40/DnaJ family. DnaJB6 and DnaJB8 are both particularly well capable of counteracting protein aggregation and toxicity mediated by protein aggregation. As described in PCT/NL2008/050207, DnaJB8 was found to be the most effective chaperone as identified in the elaborate study. At equal expression levels, DnaJB8 was found to reduce the amounts of aggregates to a much higher extent than other Hsp family members. At equal amounts of Hsp-encoding nucleic acids, the presence of a nucleic acid encoding DnaJB8 results in more protein aggregate reduction as compared to nucleic acid encoding other Hsp proteins. Therefore, a substance that is capable of enhancing the amount and/or activity of DnaJB8, or a functional part, derivative and/or analogue thereof, is particularly suitable for counteracting protein aggregation. The amount of DnaJB8 in a cell (also called the protein level of DnaJB8 in a cell) is defined herein as the amount of DnaJB8 protein and/or the amount of a functional part, derivative and/or analogue of DnaJB8 within a cell. Such amount is preferably expressed as µg DnaJB8 /cell. The present invention provides amongst other things evidence for the role of (de)acetylation events in modulating the function of DnaJB6 and DnaJB8, or a functional part, derivative or analogue thereof, in counteracting protein aggregation. The invention furthermore provides means and methods to specifically modulate these (de)acetylation events of DnaJB6 and/or DnaJB8 such that the efficacy of DnaJB6 and/or DnaJB8, or the efficacy of a functional part, functional derivative or functional analogue thereof, is enhanced for counteracting diseases associated with protein aggregation.

It is thus advantageous to influence the activity of DnaJB6 and/or DnaJB8, so that a large effect on protein aggregation is obtained. Further provided is therefore a deacetylase, nucleic acid molecule, compound or use according to the invention, wherein said heat shock protein comprises DnaJB6 or DnaJB8, or a functional part, functional derivative or functional analogue thereof. In most applications, the activity of DnaJB6 and/or DnaJB8 is preferably increased in order to decrease protein aggregation.

As used herein, the term analogue refers to an isoform of a DnaJB8 or DnaJB6 protein, which is for instance isolated from vertebrates such as from mouse, bovine, or chicken. An isoform can also be provided by, for example, conservative amino acid substitution.
The term derivative refers to a modified form of a DnaJB8 or DnaJB6 protein, including but not limited to a glycosylated form and/or a pegylated form, which may improve the pharmacological properties of a protein drug and may also expand its half life. The terms "DnaJB8 derivative" and "DnaJB6 derivative" embrace DnaJB8 and DnaJB6 proteins that are modified such that their functionality is increased and/or that are modified such that they have become more stable as compared to wild type DnaJB8 and DnaJB6.
A functional part of DnaJB8 or DnaJB6 is defined herein as a part which has the same properties in kind, not necessarily in amount. A functional part, derivative or analogue of DnaJB8 or DnaJB6 has the same capability of counteracting protein aggregation as DnaJB8 or DnaJB6, albeit not necessarily to the same extent.

The present invention furthermore provides the insight that histone deacetylase-4 (HDAC-4), or a functional part or functional derivative thereof, is particularly suitable for increasing the anti-protein aggregation activity of Hsp40/DnaJ heat shock proteins. As described above, histone deacetylases (HDACs) are enzymes that are known to catalyze the acetylation and deacetylation, respectively, of proteins at lysine residues. There are 3 different categories of HDAC: a) Class I consists of the yeast Rpd3-like proteins (HDAC1, HDAC2, HDAC3, and HDAC8); b) Class II consists of the yeast Hda1-like proteins (HDAC4, HDAC5, HDAC6, HDAC7, HDAC9, and HDAC10); c) Class III comprises the yeast Sir2-like proteins. HDAC-4 thus belongs to class II. The capability of HDAC-4 to increase the anti protein aggregation activity of Hsp40/DnaJ heat shock proteins was not known before the present invention. Now that the invention has provided this insight, HDAC-4 is thus preferably used for influencing the activity of Hsp40/DnaJ heat shock proteins.

A preferred embodiment therefore provides a deacetylase, nucleic acid molecule, compound or use according to the invention, wherein said deacetylase is HDAC-4, or a functional part or functional derivative thereof.

A functional part of HDAC-4 is defined herein as a part which has the same properties in kind, not necessarily in amount. A functional part of HDAC-4 also has the capability of enhancing anti protein aggregation activity of Hsp40/DnaJ heat shock proteins, preferably DnaJB8 and/or DnaJB6, albeit not necessarily to the same extent as HDAC-4. The term "functional derivative of HDAC-4" refers to a modified form of a HDAC-4 protein, including but not limited to a glycosylated form and/or a pegylated form, which may improve the pharmacological properties of a protein drug and may also expand its half life. The term "HDAC-4 derivative" embraces HDAC-4 proteins that are modified such that their functionality is increased and/or that are modified such that they have become more stable as compared to wild type HDAC-4. A functional derivative of HDAC-4 also has the capability of enhancing anti protein aggregation activity of Hsp40/DnaJ heat shock proteins, preferably DnaJB8 and/or DnaJB6, albeit not necessarily to the same extent as HDAC-4.

The invention thus provides a method for influencing an activity of a heat shock protein which is a member of the Hsp40/DnaJ family, the method comprising contacting said heat shock protein with HDAC-4 or a functional part or a functional derivative thereof.
Of course, it is also possible to use a nucleic acid molecule encoding HDAC-4 or a functional part or a functional derivative thereof. After administration of such nucleic acid to a cell, the cell's machinery will translate the encoded HDAC-4 or functional part or functional derivative thereof. After said HDAC-4 or functional part or functional derivative has been produced, it will influence the activity of a Hsp40/DnaJ heat shock protein.
In yet another embodiment, a compound capable of enhancing the amount and/or activity of HDAC-4 or a functional part or a functional derivative thereof in a cell is used. This way, an activity of a Hsp40/DnaJ heat shock protein is indirectly influenced. Preferably, a compound capable of enhancing the amount and/or activity of endogenous HDAC-4 in a cell is used. Administration of said compound results in an increased amount and/or activity of HDAC-4. As a result, the effect of HDAC-4 upon a Hsp40/DnaJ heat shock protein is enhanced. Said compound capable of enhancing the amount and/or activity of HDAC-4 or a functional part or a functional derivative thereof for instance comprises a compound capable of enhancing expression of said HDAC-4 or functional part or functional derivative. Alternatively, or additionally, said compound comprises an activator of HDAC-4 or a functional part or a functional derivative thereof.

Further provided is therefore a method for counteracting and/or preventing protein aggregation, comprising contacting a Hsp40/DnaJ heat shock protein with HDAC-4, or with a functional part or functional derivative of HDAC-4, or with a compound capable of enhancing the amount and/or activity of HDAC-4 in a cell, or with a nucleic acid molecule encoding HDAC-4 or encoding a functional part or functional derivative of HDAC-4. Said heat shock protein preferably comprises DnaJB6 or DnaJB8, or a functional part, derivative or analogue thereof.

In one preferred embodiment use is made of HDAC-4, and/or a functional part or functional derivative of HDAC-4, and/or a compound capable of enhancing the amount and/or activity of HDAC-4 in a cell, and/or a nucleic acid molecule encoding HDAC-4, and/or a nucleic acid molecule encoding a functional part or functional derivative of HDAC-4, for the preparation of a medicament or prophylactic agent. Such medicament or prophylactic agent is particularly suitable for counteracting and/or preventing a disorder associated with protein aggregation.

Further provided is therefore a use of HDAC-4, or a use of a functional part or functional derivative of HDAC-4, or a use of a compound capable of enhancing the amount and/or activity of HDAC-4 in a cell, or a use of a nucleic acid molecule encoding HDAC-4 or a use of a nucleic acid molecule encoding a functional part or functional derivative of HDAC-4, for the preparation of a medicament or prophylactic agent for counteracting and/or preventing a disorder associated with protein aggregation. In one preferred embodiment said disorder is associated with polyglutamine-mediated protein aggregation. More preferably said disorder is selected from Huntington's disease, Dentatorubral-pallidoluysian atrophy (DRPLA), X-linked spinal and bulbar muscular atrophy (SBMA) and/or spinocerebellar ataxias (SCA).
Methods for counteracting and/or preventing a disorder associated with protein aggregation are of course also provided. The invention thus provides a method for counteracting and/or preventing a disorder associated with protein aggregation, comprising administering to a subject in need thereof a therapeutically effective amount of HDAC-4, or a functional part or functional derivative of HDAC-4, or a compound capable of enhancing the amount and/or activity of HDAC-4 in a cell, or a nucleic acid molecule encoding HDAC-4 or encoding a functional part or functional derivative of HDAC-4. As said before, said disorder is preferably associated with polyglutamine-mediated protein aggregation. More preferably said disorder is selected from Huntington's disease, Dentatorubral-pallidoluysian atrophy (DRPLA), X-linked spinal and bulbar muscular atrophy (SBMA) and/or spinocerebellar ataxias (SCA).

In another aspect, the invention provides a use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for counteracting and/or preventing aggregation of a protein *in vitro* in the presence of a Hsp40/DnaJ heat shock protein.
Aggregation of proteins into insoluble intracellular complexes is a common problem in the production of proteins such as for instance therapeutic proteins. Therapeutic proteins are proteins that are engineered in the laboratory for pharmaceutical use and often comprise a recombinant protein. Therapeutic proteins are used to treat patients suffering from many conditions, including, but not limited to, cancer, Gaucher's disease, diabetes, anaemia, and haemophilia. Major therapeutic proteins comprise monoclonal antibodies, interferon, and erythropoietin. Other therapeutic proteins comprise insulin, blood clotting factors, vaccine antigens and soluble proteins including but not limited to growth hormones and interleukins. Aggregation of proteins during or after production involve, amongst other things, the risk of inability to manufacture said product, loss of biological activity such as loss of potency, and enhanced immunogenicity of said product. Enhanced immunogenicity is for instance caused by the high molecular weight of the aggregate and/or the fact that an aggregate displays repetitive epitopes.

Other areas that require the production of proteins, and especially purified proteins, and which will benefit from a reduction of aggregate formation include structural proteomics and the development and production of *in vitro* assays such as enzyme-linked immunoabsorbant assays and protein activity assays.

Proteins, including therapeutic proteins, are expressed in either prokaryotic or eukaryotic cells, including cells from lower eukaryotes such as *Saccharomyces cerevisiae*, and *Pichia pastoris*. Proteins can be expressed in primary cells, such as oocytes, fibroblasts and kerotinocytes. Alternatively, they can be produced in cell lines including but not limited to mammalian cell lines such as Chinese hamster ovary cells, HEK 293 cells, COS-7 cells, HeLa cells, Vero cells, MCF7 cells, Madine Darbey canine kidney cells, and PER.C6 cells. Furthermore, proteins can be produced in whole organisms, including but not limited to a plant species such as *Lemna gibba* and *Lemna minor*, Nicotiana species, and Arabidopsis species, and animal species such as *Mus musculus* and Bos *bovine*.

In a preferred aspect, the invention provides a use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for counteracting and/or preventing aggregation of a protein in the presence of a Hsp40/DnaJ heat shock protein. In one embodiment, said protein aggregation is counteracted and/or prevented *in vitro*. In a preferred embodiment, said protein aggregation is counteracted and/or prevented in a mammalian cell comprising a Hsp40/DnaJ heat shock protein.

The invention furthermore provides a use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for counteracting and/or preventing aggregation of a protein of interest in the presence of a Hsp40/DnaJ heat shock protein, whereby counteracting and/or preventing aggregation results in enhanced recovery of said protein of interest. Optimizing the levels of soluble protein is an attractive strategy to increase pure and active protein yield compared to recovering highly expressed protein in aggregated form. Recovery of aggregated proteins is usually poor and often affects the integrity and activity of the recovered protein. In addition, purification of over-expressed soluble proteins is faster and cheaper than obtaining it from aggregated forms.

HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, is of course preferably used in an environment wherein a Hsp40/DnaJ heat shock protein is also present. Said heat shock protein preferably comprises DnaJB6 and/or DnaJB8, or a functional part, derivative or analogue of DnaJB6 and/or DnaJB8.

According to the present invention, HDAC-4 is particularly well capable of increasing the activity of DnaJB6 and/or DnaJB8. This is advantageous, since DnaJB6 and DnaJB8 are particularly well capable of counteracting protein aggregation. Moreover, DnaJB6 is ubiquitously present in mammals. Since DnaJB6 and DnaJB8 are both particularly well capable of counteracting protein aggregation and toxicity mediated by protein aggregation, the use of HDAC-4 or a functional part or derivative thereof is advantageous because it is particularly suitable for counteracting protein aggregation via its effect on DnaJB6 and/or DnaJB8. HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, is thus preferably used in order to increase the activity of DnaJB6 and/or DnaJB8 in a cell. Further provided is therefore a use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for increasing the activity of DnaJB6 and/or DnaJB8 in a cell.
In one preferred embodiment the activity of DnaJB6 and/or DnaJB8 is increased in a cell *in vitro.* In another preferred embodiment, however, the activity of DnaJB6 and/or DnaJB8 is increased *in vivo*. For instance, a subject suffering from, or at risk of suffering from, protein aggregation is provided with HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, in order to increase the activity of DnaJB6 and/or DnaJB8 in the subject. The increased activity of DnaJB6 and/or DnaJB8 in the subject will result in enhanced counteraction and/or prevention of protein aggregation. Further provided is therefore a method for increasing the activity of DnaJB6 and/or DnaJB8 in a subject, comprising administering to a subject in need thereof a therapeutically effective amount of HDAC-4, or a functional part or functional derivative of HDAC-4, or a compound capable of enhancing the amount and/or activity of HDAC-4 in a cell, or a nucleic acid molecule encoding HDAC-4 or encoding a functional part or functional derivative of HDAC-4.
A use of any of the above mentioned compounds for the preparation of a medicament or prophylactic agent for increasing the activity of DnaJB6 and/or DnaJB8 of an individual is also provided. One embodiment thus provides a use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for the preparation of a medicament or prophylactic agent for increasing the activity of DnaJB6 and/or DnaJB8 of an individual.

HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, is administered to an individual by any method known in the art, for instance, but not limited to, oral administration and/or injection, for instance infusion. Emerging methods to deliver pharmaceutical substances comprise controlled delivery technologies, including local delivery technologies, needle-free systems, and pulmonary inhaler systems, which can also be used for administering HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4. Said compounds are preferably administered together with a pharmaceutically acceptable carrier, diluent or excipient.

HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4 is for instance directly administered to a cell using any known method, such as for instance injection and/or electroporation. Nucleic acid encoding HDAC-4 or a functional part or a functional derivative thereof is for instance administered to a cell using a plasmid, for instance in a virosome, and/or using a viral vector such as for instance an adenoviral, lentiviral or retroviral vector. In one embodiment a nucleic acid encoding HDAC-4 or a functional part or functional derivative thereof is administered to a cell using lipoplexes. A particularly preferred nucleic acid expression unit comprises a plasmid, which preferably comprises a nucleic acid molecule comprising a promoter, a nucleic acid sequence encoding HDAC-4 or a functional part or functional derivative thereof and, optionally, a marker gene.
Said promoter region preferably comprises regulatory sequences that control the expression from said expression unit. Suitable promoter sequences are known in the art, including, but not limited to, promoter sequences from a virus such as cytomegalovirus (CMV), or a promoter region from a housekeeping gene such as beta-actin. Alternatively, a neuron-specific promoter sequence is used to drive expression of HDAC-4, or a functional part or functional derivative thereof in neuronal cells. Examples of neuron-specific promoter sequences comprise promoter sequences of Microtubule-Associated Protein 1B Gene, neurofilament gene, gonadotropin-releasing hormone gene, and synapsin I gene.
Said termination sequences for instance comprise termination sequences, including a poly(A) signal, from the HDAC-4 gene. Alternatively, said termination sequences may be derived from other genes, including but not limited to growth hormone gene and/or the gastrin gene.
A marker gene preferably comprises a selectable marker of which the level of expression or activity can be quantified. Marker genes are known in the art and include LacZ encoding β-galactosidase, jellyfish green fluorescent protein gene, chloramphenicol acetyltransferase gene, alkaline phosphatase, and luciferase. Variants of these proteins, such as yellow fluorescent protein gene and variants with a shortened or prolonged half-life, can furthermore be used as marker gene.

Further provided is a pharmaceutical composition comprising HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4. Said pharmaceutical composition optionally comprises a pharmaceutical acceptable carrier, diluent or excipient.
The pharmaceutical composition may be presented in any form, for example as a tablet, as an injectable fluid or as an infusion fluid etc. Moreover, said pharmaceutical composition can be administered via different routes, for example intravenously, rectally, bronchially, or orally.
It is clear for the skilled person, that preferably an effective amount is delivered.
The compositions may optionally comprise pharmaceutically acceptable excipients, stabilizers, activators, carriers, permeators, propellants, desinfectants, diluents and preservatives. Suitable excipients are commonly known in the art of pharmaceutical formulation and may be readily found and applied by the skilled artisan.
For oral administration, a pharmaceutical composition according to the invention is, for example, administered in solid dosage forms, such as capsules, tablets (preferably with an enteric coating), and powders, or in liquid dosage forms, such as elixirs, syrups, and suspensions. A pharmaceutical composition according to the invention can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable colour, taste, stability, buffering capacity, dispersion or other known desirable features are red iron oxide, silica gel, sodium lauryl sulphate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance.
In a preferred embodiment a pharmaceutical composition according to the invention is suitable for oral administration and comprises an enteric coating to protect the composition from the adverse effects of gastric juices and low pH. Enteric coating and controlled release formulations are well known in the art. Enteric coating compositions in the art may comprise of a solution of a watersoluble enteric coating polymer mixed with the active ingredient(s) and other excipients, which are dispersed in an aqueous solution and which may subsequently be dried and/or pelleted. The enteric coating formed offers resistance to attack of the active ingredient(s) by atmospheric moisture and oxygen during storage and by gastric fluids and low pH after ingestion, while being readily broken down under the alkaline conditions which exist in the lower intestinal tract.

The invention furthermore provides the insight that the SSF-SST region in the C-terminal domain of Hsp40/DnaJ heat shock proteins, such as for instance DnaJB6 and DnaJB8, is involved in their capability of counteracting protein aggregation via (de)acetylation. The SSF-SST region (also called herein an SSF-SST box sequence) is a serine-rich region with no obvious secondary structure or homology to any known domain in the Pfam database.

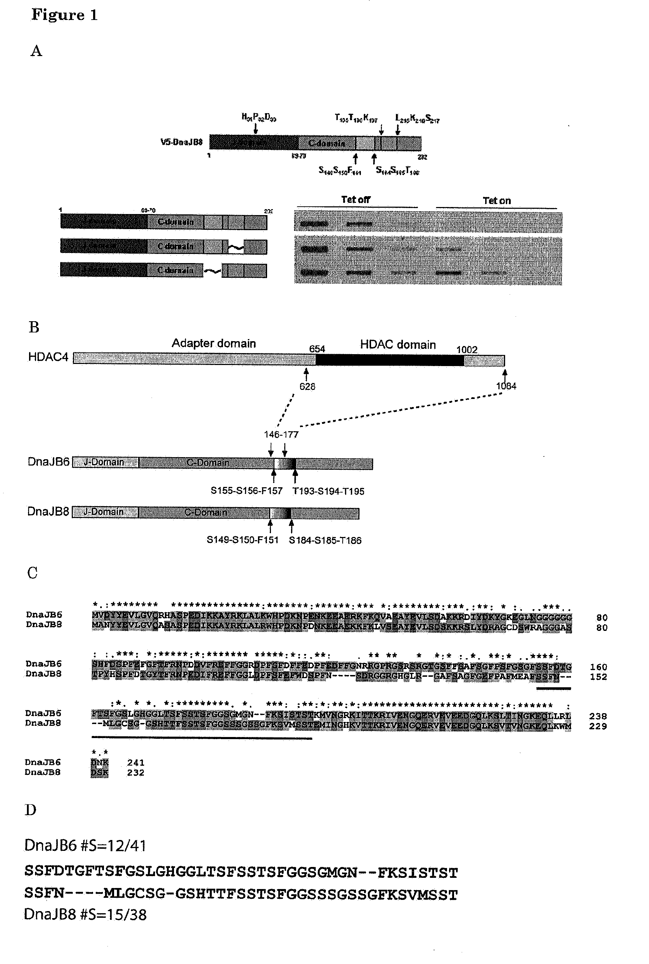

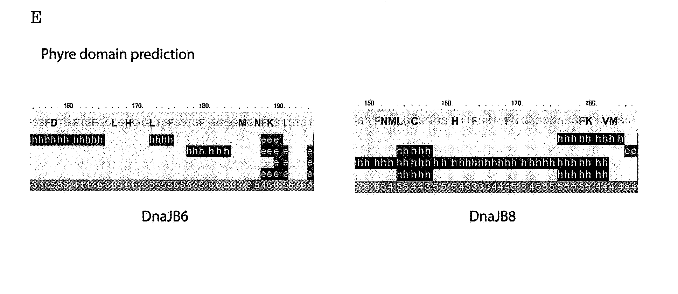

In Figure 1 the SSF-SST regions within the DnaJB8 and DnaJB6 sequences are shown.
According to the present invention, interaction with acetylase and/or deacetylase and a Hsp40/DnaJ heat shock protein requires the presence of a SSF-SST region and influences the anti protein aggregation activity of said heat shock protein. A compound such as for instance HDAC-4 which is capable of deacetylating a Hsp40/DnaJ heat shock protein via its SFF-SST domain is capable of increasing the heat shock protein's function. Now that this insight has been provided, a method is provided for increasing the anti protein aggregation activity of a Hsp40/DnaJ heat shock protein, comprising deacetylating said heat shock protein via its SSF-SST region. Said heat shock protein preferably comprises DnaJB6 and/or DnaJB8. Said heat shock protein is preferably deacetylated using a deacetylase, preferably an HDAC, or a nucleic acid encoding said deacetylase or a compound capable of enhancing the amount and/or activity of said deacetylase in a cell. In one embodiment said deacetylase comprises HDAC-4 or a functional part or a functional derivative thereof. A use of a deacetylase or a nucleic acid encoding a deacetylase or a compound capable of enhancing the amount and/or activity of a deacetylase in a cell, for the preparation of a medicament or prophylactic agent for deacetylating said heat shock protein via its SSF-SST region is also herewith provided.

Now that the invention has provided the insight that a heat shock protein's SSF-SST region is required for counteracting protein aggregation, it has become possible to design shorter heat shock variants which still comprise the SSF-SST region and which are still capable of counteracting protein aggregation. Such shorter variants are called herein functional parts of a heat shock protein capable of counteracting protein aggregation. A preferred embodiment provides a use of a functional part of DnaJB6 or DnaJB8, comprising at least amino acid residues 70-171 which comprises most of the SSF-SST region of DnaJB6 or DnaJB8, for counteracting protein aggregation, preferably *in vitro.* Said functional part preferably comprises at least 60%, more preferably at least 70%, more preferably at least 80% of the SSF-SST region of DnaJB6 or DnaJB8. Said functional part preferably constitutes the C-terminal part of DnaJB6 or DnaJB8. In one embodiment a functional part of DnaJB6 or DnaJB8, comprising at least 60%, preferably at least 70%, more preferably at least 80% of the SSF-SST region of DnaJB6 or DnaJB8, is used for the preparation of a medicament for counteracting a disorder associated with protein aggregation, preferably Huntington's disease, Dentatorubral-pallidoluysian atrophy (DRPLA), X-linked spinal and bulbar muscular atrophy (SBMA) and/or spinocerebellar ataxias (SCA). In one embodiment said functional part comprises the whole SSF-SST region of DnaJB6 or DnaJB8.

The invention is further explained in the following examples. These examples do not limit the scope of the invention, but merely serve to clarify the invention.

### Table 1

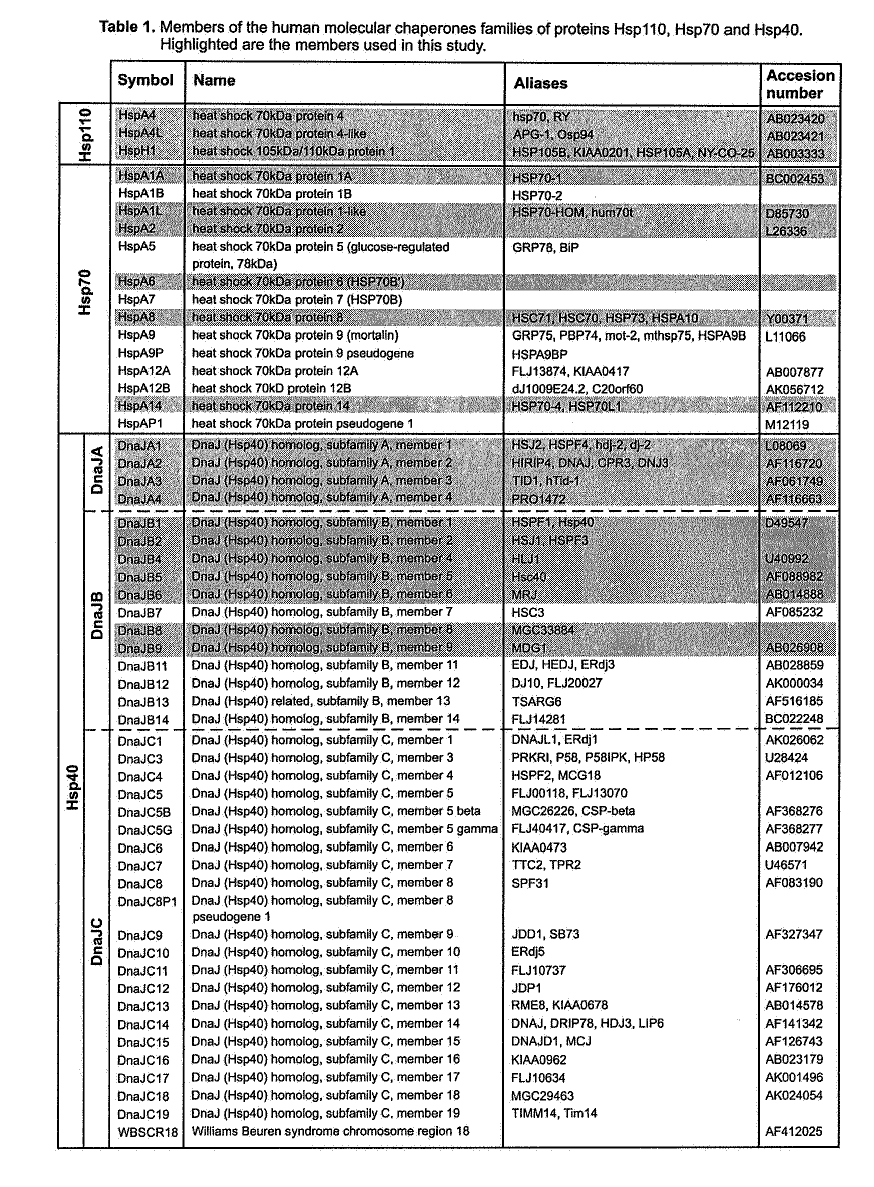

### Examples

### Materials and methods

**Cell culture and transient infections.** Flp-In T-Rex HEK293 cells (Human embryonic kidney 293) stably expressing the tetracycline (tet) repressor were obtained from Invitrogen. Cells were cultured in DMEM (Gibco) supplemented with 10% foetal bovine serum (Sigma) and 100 units/ml penicillin and 100µg/ml streptomycin (Invitrogen). 5 µg/ml Blasticidine (Sigma) and 100 µg/ml of Zeocin (Invitrogen) were added to the cultures. Cultures were maintained at 37°C and 5% CO2 in a humidified incubator. For transient transfections, cells were grown to 50-60% confluence in 35 mm-diameter dishes coated with 0.001% of poly-L-lysine (Sigma) and on coated coverslips for confocal microscopy analyses. Cells were transfected with a total of 1 µg of DNA using Lipofectamine (Gibco) according to the manufacturer instructions.

**Plasmids.** The construction of pHDQ119-EYFP driving the expression of a fragment of exon-1 of huntingtin fused to the enhanced yellow fluorescent protein was previously described (Rujano et al., 2006). The construction of the tetracycline inducible HSP overexpression plasmids is described by Hageman et al. (manuscript in preparation). Briefly, first the V5 sequence containing a Kozak initiation codon and lacking a stop codon was inserted in pcDNA5/FRT/TO by oligo cloning. Subsequently, the coding sequence of each gene was amplified and the PCR products where purified, cleaved with the respective enzymes and ligated in frame in the pcDNA5/FRT/TO-V5 vector cleaved with the same set of enzymes. Presence of the correct insert was verified using DNA sequencing. Expression of the proteins at the expected molecular mass was verified by Western blot analysis against the V5-tag.

**HDAC inhibition.** HEK-293 cells cotransfected with EYFP-HDQ119 and DnaJB6 or DnaJB8, were treated with the following HDAC inhibitors: Trichostatin A (100 and 500 nM); Butyrate (0,5 and 1 mM); Tubacin (3 and 5 □M) and Niltubacin (inactive analog of Tubacin, 3 and 5 µM). Cell extracts prepared 36 hours after treatment were processed for filter trap essays and western blotting as described before. Increase in acetylated tubulin and histones as detected by western blotting was used as test for functionality.

**Cell extracts and sample preparation**. 24 hours after transfection cells were recovered by trypsinization, pelleted and resuspended in 1 ml of PBS. The cell suspension was centrifuged at 6000 rpm for 5 min at RT and the pellet was resuspended in 75 µl of RIPA buffer containing 2% SDS supplemented with protease inhibitors and sonicated. Protein content was determined with the DC protein essay (Bio-RAD). Western blot samples were prepared at a final concentration of 1µg/µl in SDS-PAGE loading buffer and heated for 5 min at 100 °C. Filter trap samples were prepared at a final concentration of 100 ng/µl, 20 ng/µl and 4 ng/µl in FTA buffer (10 mM Tris-Cl pH 8.0, 150 mM NaCl and 50 mM dithiothreitol) + 2% SDS and heated for 5 min at 100 °C. Samples were used immediately or kept frozen at -20°C.

**Immunoprecipitation.** Cells from a 35mm dish were washed twice with PBS and lysed in 500 µl RIPA (25 mM Tris-HCl pH 7.6, 150 mM NaCl, 1% NP-40, 1% sodium deoxycholate, 0.1% SDS). Lysis was completed by passing the extracts five times through a 21-gauge needle. Subsequently, cell extracts were centrifuged twice at 10,000 G for 10 minutes at 4°C and the supernatant transferred. Lysates were cleared with agarose A/G beads for 1 hour at 4°C in a rotator. In the mean time, 0.5µg V5 anti-body was incubated with 10µl of agarose A/G plus agarose beads (Santa Cruz Biotechnology) and 50 µl RIPA for 1 hour at 4°C. Thereafter, samples were centrifuged at 1000g for 30 seconds and the supernatant was transferred to a new tube together with 50 µl of the preincubated antibody and incubated for 1 more hour in a rotator. Thereafter, the beads were washed twice with RIPA and twice with PBS. After the final wash, 30 µl of RIPA was added together with 30 µl Laemmli sample buffer and boiled for 5 minutes. 10 µl of the extract was loaded and subjected for Western blot analysis.

**Western blot analysis**. Equal amounts of protein were loaded on 10% or 12,5% SDS-PAGE gels. Proteins were transferred onto nitrocellulose membranes and probed with monoclonal anti-GFP antibody GL-8 (Clontech) at a 1:5000 dilution and monoclonal anti-V5 antibody (Invitrogen) at a 1:5000 dilution. GAPDH was used a loading control and was detected with a monoclonal antibody (RDI Research Diagnostics) at 1:1000 dilution. Blots were incubated with HRP-conjugated anti-mouse secondary antibody (Amersham) at 1:5000 dilution, and visualization was made using enhanced chemiluminescence and Hyperfilm (ECL, Amersham).

**Filter trap assay**. Filter trap assay was performed based on the protocol described by Carra et al. (2005). Briefly, 10, 2 and 0.4 µg of protein extracts were applied onto a 0,2 µm pore Cellulose Acetate membrane prewashed with FTA + 0.1% SDS. Mild suction was applied and the membrane was washed 3 times with the same buffer. Aggregated proteins trapped in the membrane were probed with monoclonal anti-GFP antibody GL-8 (Clontech) at a 1:5000 dilution and monoclonal anti-V5 antibody (Invitrogen) at a 1:5000 dilution followed by HRP-conjugated anti-mouse secondary antibody (Amersham) at 1:5000 dilution, and visualization was performed using enhanced chemiluminescence and Hyperfilm (ECL, Amersham). For quantitative analysis, relative intensity of the bands was measured using GelPro Analyzer 4.5 gel analyzer software.

**Native PAGE**. 24 hours after transfections cell extracts were prepared in non-denaturing lysis buffer (100 mM TRIS pH 7,5, 150 mM NaCl, 0.1% triton X-100, protease inhibitors) and loaded into 6-12% native PAGE. Gels were run at 120 V for 3 hours in native-running buffer (Tris-base 25 mM, Glycine 192 mM). Proteins were transferred onto nitrocellulose membranes and probed with mouse anti-V5 antibody (Invitrogen) at a 1:5000 dilution. Blots were subsequently incubated with HRP-conjugated anti-mouse secondary antibody (Amersham) at 1:5000 dilution and visualization was performed with Enhanced Chemiluminescence and Hyperfilm (ECL, Amersham).

**Sucrose gradients**. Sucrose gradients were prepared as follows: 10%, 20%, 40%, 60% and 80% sucrose solutions were prepared in 10 mM Tris-HCL, 50 mM NaCl and 5mM EDTA and columns (polyallomer, Beckman) were poured in 4500 µl of total volume. Cells from a 35 mm dish were washed twice with PBS and lysed in 200 µl Lysis buffer (150mM NaCl, 0.5% NP-40, 1.5 mM Mg2Cl). 150µl lysis buffer was loaded on the column and centrifuged for 18 hour at 100,000 g. Fractions were taken in aliquots of 350 µl and precipitated with 25% tri chloroacetic acid. Samples were incubated on ice for 30 minutes and centrifuged for 15 minutes at 12,000 g at 4°C. the precipitate was washed twice with 500 µl icecold acetone and dried. Finally, the pellets were dissolved in 60 µl 1%SDS, 0.1M NaOH and boiled for 5 minutes. 10 µl of the extract was loaded and subjected for Western blot analysis.

### Results

We previously identified DnaJB8 and its relative DnaJB6 as superior inhibitors of aggregation of proteins (patent application PCT/NL2008/050207) and that the C-terminal domain was most critical for this activity. In search for the functional domain within the C-terminus of DnaJB8, we generated several DnaJB8 and DnaJB6 mutants some of which are depicted in Figure 1. Clearly, a near to complete loss of function was seen after deletion the SSF-SST region, wheras e.g deletion of the TTK-LKS domain, typical for DnaJB6 and DnaJB8 only mariginally effected their activity.

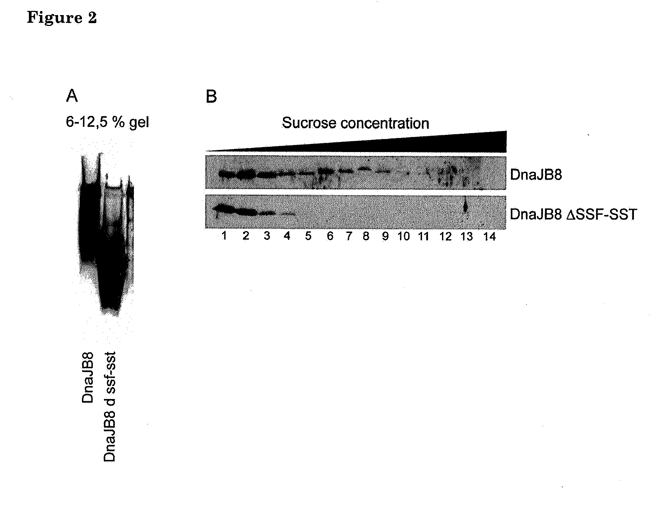

We next analysed the migration of the wildtype DnaJB8 and the SSF-SST mutant on non-denaturing gels and sucrose gradients. Clearly, wildtype DnaJB8 forms oligomeric complexes and after deletion of the SSF-SST domain the native protein migrates more rapidly (Figure 2). This implies that the SSF-SST deletion causes disruption of structure of DnaJB8 so that it can no longer undergo extensive post-translational modifications and/or that affects its ability to oligomerise.

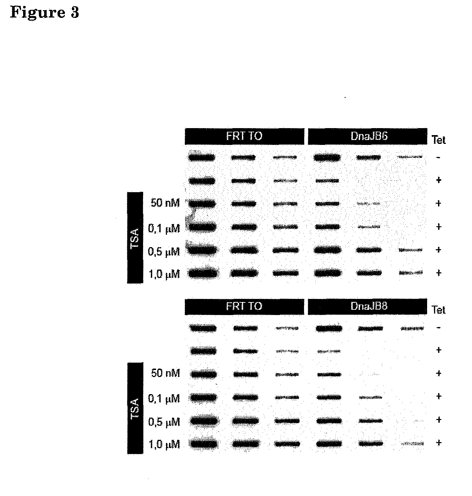

We speculated that HDAC inhibition might interfere with the (tertiarty or oligomeric) structure and function of DnaJB8. To first test DnaJB8 function in relation to HDAC activities, we first tested the effects of a variety of HDAC inhibitors on the ability of DnaJB8 to suppress HDQ119 aggregation. Hereto cells were co-transfected with HDQ119 and tet-inducible DnaJB8 or an empty plasmid (FRT TO) in the absence or presence of different concentrations of trichostatin A (TSA), a global inhibitor of HDAC (Figure 3). TSA slightly enhanced HDQ119 aggregation in cells transfected with the empty vector. More spectacular, however, was the effect on cells expressing DnaJB6 or DnaJB8: here, TSA resulted in a concentration dependent loss of the abililty of DnaJB8 and DnaJB6 to reduce HQ119 aggregation. In fact, treatment with 0.5 µM TSA completely annihilated DnaJB8 and DnaJB6 activity.

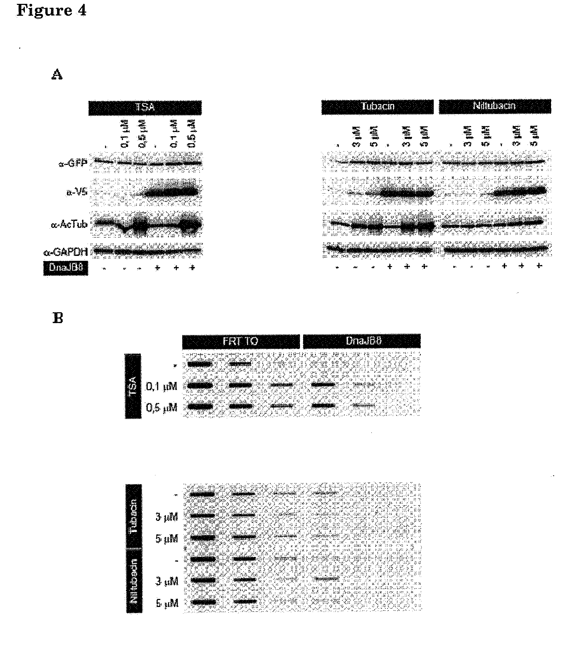

There are 3 different categories of HDAC: a) Class I consists of the yeast Rpd3-like proteins (HDAC1, HDAC2, HDAC3, and HDAC8); b) Class II consists of the yeast Hda1-like proteins (HDAC4, HDAC5, HDAC6, HDAC7, HDAC9, and HDAC10); c) Class III comprises the yeast Sir2-like proteins.
TSA is a very potent but rather specific inhibitor of all HDACs. Since HDAC6 has been implicated in Huntington's disease (Dompierre JP, Godin JD, Charrin BC, Cordelières FP, King SJ, Humbert S, Saudou F., Histone deacetylase 6 inhibition compensates for the transport deficit in Huntington's disease by increasing tubulin acetylation. 1: J Neurosci. 27 (2007) 3571-3583) we therefore first tested whether Tubacin, a specifical inhibitor of HDAC 6 that e.g. is known to deacytylate tubulin can inhibit DnaJB6/8 activity. As can be seen in Figure 4A, both TSA and Tubacin are equally effective in increasing tubulin acetylation; the specificity of the Tubacin effect was further demonstrated by the lack of the effect of Nitubacin, a non-effective analogue of Tubacin. Interestingly, unlike TSA Tubacin did not result in inhibition of DnaJB8 and DnaJB6 activity on HD 119Q aggregation (Figure 4B). This shows that HDAC6 is not involved in regulating DnaJB6 and DnaJB8 activity.

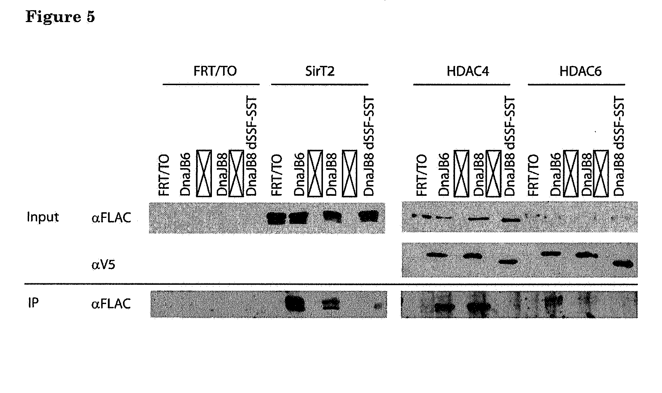

Although additional work with other more or less specific inhibitors in ongoing, lack of real specific inhibitors prompted us to more directly test whether various HDAC members could directly bind to DnaJB8. Hereto, we executed immunoprecipitations with a (yet limited) number of HDAC members. As can be seen in Figure 5, DnaJB8 to co-immunoprecipitated with HDAC-4, HDAC-6 (both type II) and Sirt-2 (Type III), but not with HDAC-1, -3, -5, -7, -8). Most importantly, deleting the SSF-SST domain of DnaJB8, which abolishes its tertiairy structure and function (Figure 1,2), resulted in loss of interaction between DnaJB8 and HDAC-4, HDAC-6 (both type II) or Sirt-2 (Figure 5). Given that TSA does not inhibit type III HDACs but yet inhibits DnaJB8 function (Figure 3, 4) and given the finding that HDAC-6 seems not involved in regulating DnaJB8 activity (Figure 4), the combined data show that HDAC-4 plays a prominent role in regulating DnaJB8 activity via the SSF-SST domain.

### Brief description of the drawings

**Figure 1****: The SSF-SST box within the C-domain of DnaJB8 is most crucial for inhibition of HDQ119 aggregation**. A) Filter trap assay in samples without and with overexpression of the indicated variants of DnaJB8 (Tet of/Tet on) prepared 24h after cotransfection. Samples were slot-blotted at three different concentrations (10, 2 and 0.4 ug of protein/slot) in cellulose - acetate membranes and probed with anti-GFP antibody. Expression (Tet on)) of the wild type DnaJB8 and TTK-LKS mutant substantially suppressed aggregation of poly-Q huntingtin, whereas the suppressive effect was virtually lost when overexpression the mutant DnaJB8 lacking the SSF-SST box. Similar data were obtained for DnaJB6 (data not shown). B) Proposed interaction of DnaJB6 with HDAC-4 according to Mol Cell Biol. 2005 Nov;25(22):9936-48. C) Context of the SSF-TST box within the DnaJB6 protein and the SSF-TST box in DnaJB8. SSF-SST, SSF-SST is underlined. D) SSF-TST and SSF-SST box in detail. This motif is very rich in serines. The number of serines is indicated. E) Phyre secondary domain prediction of the DnaJB6 SSF-TST and DnaJB8 SSF-SST regions. No clear secundairy structure can be derived from these predictions.
**Figure 2****: The SSF-SST box within the C-terminal domain of DnaJB8 alters the migration rate of DnaJB8 on native gels and also the distribution on sucrose gradients.** Cells transfected with either full length DnaJB8 or the SSF-SST deletion mutant were harvested under non-denaturing conditions. A) Cell lysates were run on 6-12,5% native PAGE. Proteins were transferred to nitrocellulose membranes and probed with anti-V5 antibodies for detection of the V5-tagged DnaJB8 variants. B) Proteins in cell lysates were separated in a 10-80% sucrose gradient and precipitated proteins from collected fractions were run in 10% SDS-PAGE, transferred to nitrocellulose membranes and probed with anti-V5 antibodies. Deletion of the SSF-SST box in DnaJB8 resulted in a much faster migration rate of DnaJB8 in the native gel and altered distribution in the sucrose fractions, indicative of either reduced posttranslational modifications and/or hampered formation of oligomeric structures.
**Figure 3****: Treatment of cells with the HDAC inhibitor trichostatin A (TSA) results in loss of DnaJB8 and DnaJB6 activity to inhibit HDQ119 aggregation.** Filter trap assay in samples without and with overexpression of DnaJB6 (top) or DnaJB8 (bottum) (Tet of/Tet on) for 24h in the presences of increasing concentrations of TSA. Samples were slot-blotted at three different concentrations (10, 2 and 0.4 ug of protein/slot) in cellulose - acetate membranes and probed with anti-GFP antibody. Expression (+ Tet) of DnaJB6 or DnaJB8 strongly suppressed aggregation of poly-Q huntingtin but not after HDAC inhibition with TSA.
**Figure 4****: Treatment of cells with the specific HDAC-6 inhibitor Tubacin does not result in loss of DnaJB8 activity to inhibit HDQ119 aggregation.** Panel A: Western blot showing that the HDAC inhibitors TSA and Tubacin (but not its non-functional structural analogue Nitubacin) increases the cellular content of acytelated Tubulin both in control cells or in cells expressing DnaJB8, indicating that both inhibitors equally inhibit HDAC-6. Panel B: Filter trap assay in samples without and with overexpression of DnaJB8 (+/- Tet) for 24h in the presences of TSA and Tubacin (and its non-functional structural analogue Nitubacin). Samples were slot-blotted at three different concentrations (10, 2 and 0.4 ug of protein/slot) in cellulose - acetate membranes and probed with anti-GFP antibody. Expression (+ Tet) of DnaJB8 strongly suppressed aggregation of poly-Q huntingtin. This effect was stronly reduced by TSA but not by Tubacin (or Nitubacin) excluding a major role for HDAC-6 in regulation DnaJB6 and DnaJB8 activity.
**Figure 5****: DnaJB8 interacts with HDAC-4, HDAC-6 and Sirt-2 via its SSF-SST region.** Samples co-expressing the indicated FLAG-tagged HDACs and DnaJB6 or DnaJB8 proteins were lysed 24h after transfection and immunoprecipitated using the V5 antibody. Deletion of the SSF-SST region abolished the immuno precipitation and hence the interaction.

### References

Arrasate,M., Mitra,S., Schweitzer,E.S., Segal,M.R., and Finkbeiner,S. (2004). Inclusion body formation reduces levels of mutant huntingtin and the risk of neuronal death. Nature 431, 805-810.
E. A. Bates, M. Victor, A.K. Jones, Y. Shi, and A. C. Hart, Differential Contributions of Caenorhabditis elegans Histone Deacetylases to Huntingtin Polyglutamine Toxicity. The Journal of Neuroscience, 26, 2006, 2830-2838
Bailey,C.K., Andriola,I.F.M., Kampinga,H.H., and Merry,D.E. (2002). Molecular chaperones enhance the degradation of expanded polyglutamine repeat androgen receptor in a cellular model of spinal and bulbar muscular atrophy. Hum. Mol. Genet. 11, 515-523.
Behrends,C., Langer,C.A., Boteva,R., Bottcher,U.M., Stemp,M.J., Schaffar,G., Rao,B.V., Giese,A., Kretzschmar,H., Siegers,K., and Hartl,F.U. (2006). Chaperonin TRiC promotes the assembly of polyQ expansion proteins into nontoxic oligomers. Mol. Cell 23, 887-897.
Bence,N.F., Sampat,R.M., and Kopito,R.R. (2001). Impairment of the ubiquitin-proteasome system by protein aggregation. Science 292, 1552-1555.
Carra,S., Sivilotti,M., Chavez Zobel,A.T., Lambert,H., and Landry,J. (2005). HspB8, a small heat shock protein mutated in human neuromuscular disorders, has in vivo chaperone activity in cultured cells. Hum. Mol. Genet. 14, 1659-1669.
Chai,Y.H., Koppenhafer,S.L., Bonini,N.M., and Paulson,H.L. (1999). Analysis of the role of heat shock protein (Hsp) molecular chaperones in polyglutamine disease. J. Neurosci. 19, 10338-10347.
Cheetham,M.E. and Caplan,A.J. (1998). Structure, function and evolution of DnaJ: conservation and adaptation of chaperone function. Cell Stress & Chaperones 3, 28-36.
Chuang,J.Z., Zhou,H., Zhu,M., Li,S.H., Li,X.J., and Sung,C.H. (2002a). Characterization of a brain-enriched chaperone, MRJ, that inhibits Huntingtin aggregation and toxicity independently. J. Biol. Chem. 277, 19831-19838.
Cummings,C.J., Mancini,M.A., Antalffy,B., DeFranco,D.B., Orr,H.T., and Zoghbi,H.Y. (1998). Chaperone suppression of aggregation and altered subcellular proteasome localization imply protein misfolding in SCA1. Nat. Gen. 19, 148-154.
Cummings,C.J., Sun,Y., Opal,P., Antalffy,B., Mestril,R., Orr,H.T., Dillmann,W.H., and Zoghbi,H.Y. (2001). Over-expression of inducible HSP70 chaperone suppresses neuropathology and improves motor function in SCA1 mice. Hum. Mol. Genet. 10, 1511-1518.
Dompierre JP, Godin JD, Charrin BC, Cordelières FP, King SJ, Humbert S, Saudou F., Histone deacetylase 6 inhibition compensates for the transport deficit in Huntington's disease by increasing tubulin acetylation. 1: J Neurosci. 27 (2007) 3571-3583
Fernandez-Funez,P., Nino-Rosales,M.L., de Gouyon,B., She,W.C., Luchak,J.M., Martinez,P., Turiegano,E., Benito,J., Capovilla,M., Skinner,P.J., McCall,A., Canal,I., Orr,H.T., Zoghbi,H.Y., and Botas,J. (2000). Identification of genes that modify ataxin-1-induced neurodegeneration. Nature 408, 101-106.
Frydman,J. (2001). Folding of newly translated proteins in vivo: the role of molecular chaperones. Annu. Rev. Biochem. 70, 603-647.
Guarente,L and Picard,F. Calorie Restriction- the SIR2 Connection, Cell 120 (2005) 473-482
Gurbuxani et al. Oncogene 20: 7478-7485 (2001)).
Hanai,R. and Mashima,K. (2003). Characterization of two isoforms of a human DnaJ homologue, HSJ2. Mol. Biol. Rep. 30, 149-153.
Hansson,O., Nylandsted,J., Castilho,R.F., Leist,M., Jaattela,M., and Brundin,P. (2003). Overexpression of heat shock protein 70 in R6/2 Huntington's disease mice has only modest effects on disease progression. Brain Research 970, 47-57.
Hartl,F.U. and Hayer-Hartl,M. (2002). Protein folding - Molecular chaperones in the cytosol: from nascent chain to folded protein. Science 295, 1852-1858.
Hay,D.G., Sathasivam,K., Tobaben,S., Stahl,B., Marber,M., Mestril,R., Mahal,A., Smith,D.L., Woodman,B., and Bates,G.P. (2004). Progressive decrease in chaperone protein levels in a mouse model of Huntington's disease and induction of stress proteins as a therapeutic approach. Hum. Mol. Genet. 13, 1389-1405:
Holmberg,C.I., Staniszewski,K.E., Mensah,K.N., Matouschek,A., and Morimoto,R.I. (2004). Inefficient degradation of truncated polyglutamine proteins by the proteasome. Embo Journal 23, 4307-4318.
Huen,N.Y. and Chan,H.Y. (2005). Dynamic regulation of molecular chaperone gene expression in polyglutamine disease. Biochem. Biophys. Res. Commun. 334, 1074-1084.
Hunter,P.J., Swanson,B.J., Haendel,M.A., Lyons,G.E., and Cross,J.C. (1999). Mrj encodes a DnaJ-related co-chaperone that is essential for murine placental development. Development 126, 1247-1258.
Jana,N.R., Tanaka,M., Wang,G.H., and Nukina,N. (2000). Polyglutamine length-dependent interaction of Hsp40 and Hsp70 family chaperones with truncated N-terminal huntingtin: their role in suppression of aggregation and cellular toxicity. Hum. Mol. Genet. 9, 2009-2018.
Johnston,J.A., Ward,C.W., and Kopito,R.R. (1998). Aggresomes: A cellular response to misfolded proteins. J. Cell Biol. 143, 1883-1898.
Kampinga,H.H., Kanon,B., Salomons,F.A., Kabakov,A.E., and Patterson,C. (2003). Overexpression of the cochaperone CHIP enhances Hsp70-dependent folding activity in mammalian cells. Mol. Cell Biol. 23, 4948-4958.
Kazemi-Esfarjani,P. and Benzer,S. (2000). Genetic suppression of polyglutamine toxicity in Drosophila. Science 287, 1837-1840.
Kelley,W.L. (1998). The J-domain family and the recruitment of chaperone power. Trends in Biochemical Sciences 23, 222-227.
Kobayashi,Y., Kume,A., Li,M., Doyu,M., Hata,M., Ohtsuka,K., and Sobue,G. (2000). Chaperones Hsp70 and Hsp40 suppress aggregate formation and apoptosis in cultured neuronal cells expressing truncated androgen receptor protein with expanded polyglutamine tract. J. Biol. Chem. 275, 8772-8778.
Kopito,R.R. (2000). Aggresomes, inclusion bodies and protein aggregation. Trends Cell Biol. 10, 524-530.
Kroll,K.L. and Amaya,E. (1996). Transgenic Xenopus embryos from sperm nuclear transplantations reveal FGF signaling requirements during gastrulation. Development 122, 3173-3183.
Lipinski et al. (1997) Adv Drug Deliv Rev 23: 3-25).
Michels,A.A., Kanon,B., Bensaude,O., and Kampinga,H.H. (1999). Heat shock protein (Hsp) 40 mutants inhibit Hsp70 in mammalian cells. J. Biol. Chem. 274, 36757-36763.
Michels,A.A., Kanon,B., Konings,A.W.T., Ohtsuka,K., Bensaude,O., and Kampinga,H.H. (1997). Hsp70 and Hsp40 chaperone activities in the cytoplasm and the nucleus of mammalian cells. J. Biol. Chem. 272, 33283-33289.
Mohun,T.J., Garrett,N., and Gurdon,J.B. (1986). Upstream sequences required for tissue-specific activation of the cardiac actin gene in Xenopus laevis embryos. EMBO J. 5, 3185-3193.
Nollen,E.A.A., Brunsting,J.F., Song,J.H., Kampinga,H.H., and Morimoto,R.I. (2000). Bag1 functions in vivo as a negative regulator of Hsp70 chaperone activity. Mol. Cell Biol. 20, 1083-1088.
Nollen,E.A.A., Garcia,S.M., van Haaften,G., Kim,S., Chavez,A., Morimoto,R.I., and Plasterk,R.H.A. (2004). Genome-wide RNA interference screen identifies previously undescribed regulators of polyglutamine aggregation. Proceedings of the National Academy of Sciences of the United States of America 101, 6403-6408.
Nucifora FC Jr, Sasaki M, Peters MF, Huang H, Cooper JK, Yamada M, Takahashi H, Tsuji S, Troncoso J, Dawson VL, Dawson TM, Ross CA (2001) Interference by huntingtin and atrophin-1 with CBP1-mediated transcription leading to cellular toxicity. Science 291:2423-2428
Parker JA, Arango M, Abderrahmane S, Lambert E, Tourette C, Catoire H, Néri C. Resveratrol rescues mutant polyglutamine cytotoxicity in nematode and mammalian neurons, Nat Genet. 37 (2005) 349-350
Pei,L. (1999). Pituitary tumor-transforming gene protein associates with ribosomal protein S10 and a novel human homologue of DnaJ in testicular cells. J. Biol. Chem. 274, 3151-3158.
Purva Bali, Michael Pranpat, James Bradner, Maria Balasis, Warren Fiskus, Fei Guo, Kathy Rocha, Sandhya Kumaraswamy, Sandhya Boyapalle, Peter Atadja, Edward Seto, and Kapil Bhalla, Inhibition of Histone Deacetylase 6 Acetylates and Disrupts the Chaperone Function of Heat Shock Protein 90, J. Biol. Chem., 280 (2005) 26729-26734
Rohde M, Daugaard M, Jensen MH, Helin K, Nylandsted J, Jaattela M. Members of the heat-shock protein 70 family promote cancer cell growth by distinct mechanisms. Genes Dev. 2005 Mar 1;19(5):570-82
Rujano,M.A., Bosveld,F., Salomons,F.A., Dijk,F., van Waarde,M.A., van der Want,J.J., de Vos,R.A., Brunt,E.R., Sibon,O.C., and Kampinga,H.H. (2006). Polarised Asymmetric Inheritance of Accumulated Protein Damage in Higher Eukaryotes. PLoS. Biol. 4, e417.
Rujano,M.A. and Kampinga,H.H. (2007). The HSP70 chaperone machine as guardian of the proteome: Implications for protein misfolding diseases. In Heat Shock Proteins in Biology and Medicine, J.Radons and G.Multhoff, eds. Research Signpost).
Segal et al. (1999) PNAS 96: 2758-2763
Seki,N., Hattori,A., Hayashi,A., Kozuma,S., Miyajima,N., and Saito,T. (1999). Cloning, tissue expression, and chromosomal assignment of human MRJ gene for a member of the DNAJ protein family. J. Hum. Genet. 44, 185-189.
Sittler,A., Lurz,R., Lueder,G., Priller,J., Hayer-Hartl,M.K., Hartl,F.U., Lehrach,H., and Wanker,E.E. (2001). Geldanamycin activates a heat shock response and inhibits huntingtin aggregation in a cell culture model of Huntington's disease. Hum. Mol. Genet. 10, 1307-1315.
Sparrow,D.B., Latinkic,B., and Mohun,T.J. (2000). A simplified method of generating transgenic Xenopus. Nucleic Acids Res. 28, E12.
Stenoien,D.L., Cummings,C.J., Adams,H.P., Mancini,M.G., Patel,K., DeMartino,G.N., Marcelli,M., Weigel,N.L., and Mancini,M.A. (1999). Polyglutamine-expanded androgen receptors form aggregates that sequester heat shock proteins, proteasome components and SRC-1, and are suppressed by the HDJ-2 chaperone. Hum. Mol. Genet. 8, 731-741.
Takayama,S., Bimston,D.N., Matsuzawa,S., Freeman,B.C., AimeSempe,C., Xie,Z.H., Morimoto,R.I., and Reed,J.C. (1997). BAG-1 modulates the chaperone activity of Hsp70/Hsc70. Embo Journal 16, 4887-4896.
Taylor,J.P., Tanaka,F., Robitschek,J., Sandoval,C.M., Taye,A., Markovic-Plese,S., and Fischbeck,K.H. (2003). Aggresomes protect cells by enhancing the degradation of toxic polyglutamine-containing protein. Hum. Mol. Genet. 12, 749-757.
Turner,D.L. and Weintraub,H. (1994). Expression of achaete-scute homolog 3 in Xenopus embryos converts ectodermal cells to a neural fate. Genes Dev. 8, 1434-1447.
Venkatraman,P., Wetzel,R., Tanaka,M., Nukina,N., and Goldberg,A.L. (2004). Eukaryotic proteasomes cannot digest polyglutamine sequences and release them during degradation of polyglutamine-containing proteins. Mol. Cell 14, 95-104.
Warrick,J.M., Chan,H.Y.E., Gray-Board, Chai,Y.H., Paulson,H.L., and Bonini,N.M. (1999). Suppression of polyglutamine-mediated neurodegeneration in Drosophila by the molecular chaperone HSP70. Nat. Gen. 23, 425-428.
Wyttenbach,A., Carmichael,J., Swartz,J., Furlong,R.A., Narain,Y., Rankin,J., and Rubinsztein,D.C. (2000). Effects of heat shock, heat shock protein 40 (HDJ-2), and proteasome inhibition on protein aggregation in cellular models of Huntington's disease. Proc. Natl. Acad. Sci. U. S. A 97, 2898-2903
(Wood et al. (2003) Neuropathology and Applied Neurobiology 29: 529-545).

## Claims

1. A deacetylase or a nucleic acid molecule encoding a deacetylase or a compound capable of enhancing the amount and/or activity of a deacetylase in a cell, for use as a medicament or prophylactic agent for influencing an activity of a heat shock protein which is a member of the Hsp40/DnaJ family.

2. Use of a deacetylase or a nucleic acid molecule encoding a deacetylase or a compound capable of enhancing the amount and/or activity of a deacetylase in a cell, for the preparation of a medicament or prophylactic agent for influencing an activity of a heat shock protein which is a member of the Hsp40/DnaJ family.

3. A deacetylase, nucleic acid molecule, compound or use according to claim 1 or 2, wherein said activity of said heat shock protein is enhanced.

4. A deacetylase, nucleic acid molecule, compound or use according to any one of claims 1-3, wherein said heat shock protein is capable of at least in part counteracting and/or preventing a disorder associated with protein aggregation.

5. A deacetylase, nucleic acid molecule, compound or use according to any one of claims 1-4, wherein said disorder is associated with polyglutamine-mediated protein aggregation.

6. A deacetylase, nucleic acid molecule, compound or use according to any one of claims 1-5, wherein said disorder is selected from Huntington's disease, Dentatorubral-pallidoluysian atrophy (DRPLA), X-linked spinal and bulbar muscular atrophy (SBMA) and/or spinocerebellar ataxias (SCA).

7. A deacetylase, nucleic acid molecule, compound or use according to any one of claims 1-6, wherein said heat shock protein comprises DnaJB6 or DnaJB8, or a functional part or functional derivative thereof.

8. A deacetylase, nucleic acid molecule, compound or use according to any one of claims 1-7, wherein said deacetylase is HDAC-4, or a functional part or functional derivative thereof.

9. Use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for the preparation of a medicament or prophylactic agent for counteracting and/or preventing a disorder associated with protein aggregation.

10. A method for counteracting and/or preventing a disorder associated with protein aggregation, comprising administering to a subject in need thereof a therapeutically effective amount of HDAC-4, or a functional part or functional derivative of HDAC-4, or a compound capable of enhancing the amount and/or activity of HDAC-4 in a cell, or a nucleic acid molecule encoding HDAC-4 or encoding a functional part or functional derivative of HDAC-4.

11. Use or method according to claim 9 or 10, wherein said disorder is associated with polyglutamine-mediated protein aggregation.

12. Use or method according to any one of claims 9-11, wherein said disorder is selected from Huntington's disease, Dentatorubral-pallidoluysian atrophy (DRPLA), X-linked spinal and bulbar muscular atrophy (SBMA) and/or spinocerebellar ataxias (SCA).

13. Use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for counteracting and/or preventing aggregation of a protein.

14. Use according to claim 13, wherein aggregation of a protein is counteracted and/or prevented in a mammalian cell.

15. Use according to claim 13 or claim 14, wherein counteracting and/or preventing aggregation of a protein results in enhanced recovery of said protein.

16. A method for increasing the activity of DnaJB6 and/or DnaJB8 in a subject, comprising administering to a subject in need thereof a therapeutically effective amount of HDAC-4, or a functional part or functional derivative of HDAC-4, or a compound capable of enhancing the amount and/or activity of HDAC-4 in a cell, or a nucleic acid molecule encoding HDAC-4 or encoding a functional part or functional derivative of HDAC-4.

17. Use of HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, for the preparation of a medicament or prophylactic agent for increasing the activity of DnaJB6 and/or DnaJB8 of an individual.

18. A pharmaceutical composition comprising HDAC-4, or a functional part or functional derivative of HDAC-4, or a compound capable of enhancing the amount and/or activity of HDAC-4 in a cell, or a nucleic acid molecule encoding HDAC-4 or a nucleic acid molecule encoding a functional part or functional derivative of HDAC-4, optionally further comprising a pharmaceutical acceptable carrier, diluent or excipient.
